# EUROPÄISCHE PATENTSCHRIFT

(11) **EP 0 699 094 B1**
(45) Veröffentlichungstag und Bekanntmachung des Hinweises auf die Patenterteilung: **06.08.1997**
(21) Anmeldenummer: 94915014.8
(22) Anmeldetag: 04.05.1994
(51) Int. Cl.: A61N 5/06

(54) **SCHULTERBRÄUNER**
SHOULDER BROWNER
DISPOSITIF POUR LE BRONZAGE DES EPAULES

(30) Priorität: 04.05.1993 DE 4314679
(43) Veröffentlichungstag der Anmeldung: 06.03.1996
(73) Patentinhaber: Imab Stiftung, FL-9496 Balzers (LI)
(72) Erfinder: BRÜCK, Gernot, K., NL-6432 JP Hoensbroek (NL)
(74) Vertreter: Lippert, Hans-Joachim, Dipl.-Ing.
(86) Internationale Anmeldenummer: DE9400498
(87) Internationale Veröffentlichungsnummer: WO9425109

(56) Entgegenhaltungen:
- EP-A- 0 208 309
- WO-A-88/08267

## Beschreibung

### Stand der Technik

Bei allen Sonnenbänken erfolgt die Bräunung nur von zwei Seiten und bei einigen Hochdruckbänken sogar nur von einer Seite.

Der Benutzer liegt bei dem überwiegenden Teil der Profi-Bänke auf einer Liege, die mit einer großen Zahl von UV-Niederdruckröhren ausgestattet ist. Durch diese Röhren erfolgt die Bräunung des Rückens.

Im Oberteil der Sonnenbank befindet sich ebenfalls eine große Zahl von UV-Röhren und im Gesichtsbereich eine Anzahl von Hochdruckeinheiten, bestehend aus dem Strahler, dem Reflektor und einem geeigneten Filter.

Bei diesen Gesichtsfeldern werden überwiegend Reflektorkästen eingesetzt, bei denen der Strahler entlang der Sonnenbankorientierung eingesetzt ist. In diesen Fällen verläuft die Parabolform des Reflektorkastens nur um die Längsachse des Strahlers herum. Auf diese Weise entsteht eine Abstrahlcharakteristik des Gesichtsfeldes derart, daß die Maximalintensität als langer Streifen entlang des Körpers des Benutzers verläuft. So erhält neben dem Kopf- auch der Brustbereich eine starke Bestrahlung durch die Gesichtsfelder. Da diese aber immer so angeordnet sind, daß das Zentrum der Bestrahlung auf den Kopf hin weist, geht jede über den Kopf hinausragende Bestrahlung verloren.

Durch diese Anordnung der Beleuchtungssysteme bleibt die Schulterpartie immer unberücksichtigt und wird nur durch den Teil der tangentialen Strahlung bedient. Die sich daraus ergebende Bestrahlung ist verhältnismäßig gering, so daß es zu keiner Bräunung der Schultern kommt.

### Problem

Da es nun gerade Aufgabe der Solarien ist, eine echte und realistische Urlaubsbräune zu vermitteln, wirkt der Mangel an Schulterbräunung sehr negativ, da bei der natürlichen Besonnung gerade die Schulterpartien sehr häufig überbraun werden.

Auf diese Weise kann bei dem gebräunten Menschen erkannt werden, ob er diese Bräune über eine Besonnung im Solarium erhalten hat oder ob es sich um eine Sonnenbräune handelt.

### Problemlösung

Um diesem Mangel zu begegnen, ist es notwendig, auch für eine ausreichende Schulterbräunung zu sorgen. Diese kann durch entsprechend angeordnete Bestrahlungssysteme geschehen oder eben durch die erfindungsgemäße Vorrichtung.

### Der Schulterbräuner

Beim erfindungsgemäßen Schulterbräuner werden passive und aktive Vorrichtungen unterschieden.

In allen Fällen wird das übliche Kopfkissen durch eine erfindungsgemäße Vorrichtung ersetzt, wobei die notwendigen Elemente an das Kissen angebaut werden.

Die Erfindung wird anhand der Figuren beispielsweise veranschaulicht. Es zeigen:

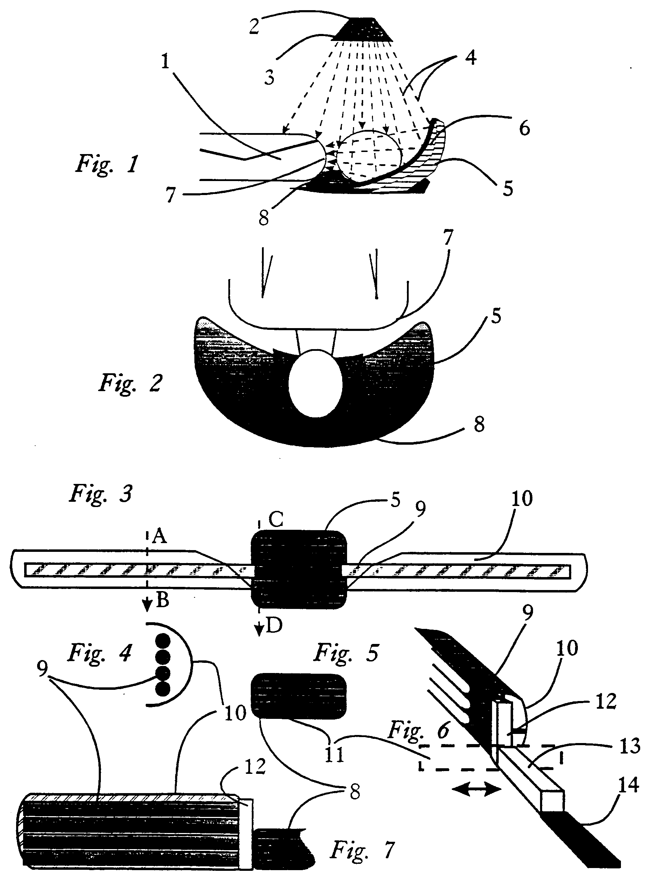

- Fig. 1: eine Seitenansicht des Gesichtsfeldes einer Sonnenbank mit einem passiven Schulterbräuner,
- Fig. 2: eine Draufsicht auf den passiven Schulterbräuner gem. Fig. 1,
- Fig. 3: eine Draufsicht auf einen aktiven Schulterbräuner mit zwei Reflektoren,
- Fig. 4: einen Schnitt in der Ebene A-B gem. Figur 3,
- Fig. 5: einen Schnitt in der Ebene C-D gem. Fig. 3,
- Fig. 6: eine aufgeschnittene perspektivische Ansicht des aktiven Schulterbräuners gem. Fig. 3,
- Fig. 7: eine senkrechte Ansicht auf den Schulterbräuner gem. Fig. 3,

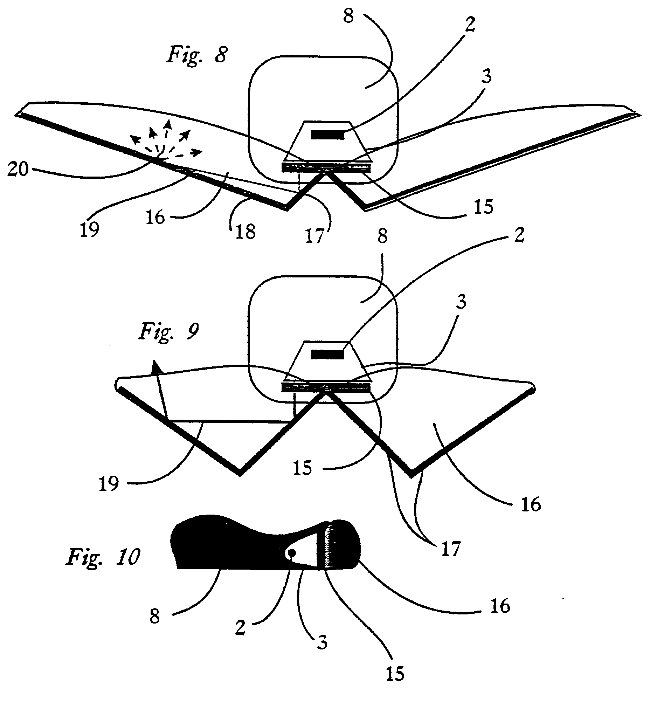

- Fig. 8: eine Draufsicht auf einen aktiven Schulterbräuner mit Reflektoren und Streulichtebenen,
- Fig. 9: eine Draufsicht auf einen aktiven Schulterbräuner mit Reflektoren und
- Fig. 10: einen Querschnitt durch einen aktiven Schulterbräuner gem. Fig. 8 oder 9.

### 1. Der passive Schulterbräuner

Um das Kissen 8 herum wird ein Kragen 5 aus einem festen Material angebracht. Die Oberfläche 6 ist hochglanzverspiegelt. Die Form wird beschrieben durch die aus den Gesichtsfeldern einfallende Strahlung 4 der zu bestrahlenden Schulterpartie 7.

Die Oberfläche 6 des Reflektorkragens 5 um das Kissen 8 herum ist jeweils so orientiert, daß das Lot senkrecht auf dem Oberflächenelement jeweils Winkelhalbierende des Winkels ist, der gebildet wird aus dem aus dem Gesichtsbräuner austretenden Strahl 4 und dem auf die Schulter 7 auftreffenden Strahl.

Erfindungsgemäß wird die überschüssige Leistung der Gesichtsbräuner 2,3, die sonst oberhalb des Kopfes verloren geht, in Nutzstrahlung für die Schulterpartie 7 umgewandelt.

### 2. Der aktive Schulterbräuner

Reicht der passive Schulterbräuner nicht aus, weil die über den Kopf hinausreichende Strahlung zu gering ist, dann empfiehlt sich der Einsatz eines aktiven Schulterbräuners. Hierbei ist erfindungsgemäß zwischen einem Niederdrucktyp und einem Hochdrucktyp zu unterscheiden.

Im ersteren Fall befindet sich im Kopfkissen ein eingearbeiteter Schacht 11, durch den ein Verbindungsgestell 13 bewegt wird. An dieses schließt sich beidseitig eine Sockelhalterung 12 an, in der jeweils einseitig gesockelte Lampen 9 eingepaßt sind. Ein Reflektor 10 aus spiegelndem Material ist dahinter zu den Schultern 7 hin angebracht.

Da sich die gesamte Leuchten-/Reflektoreneinheit 9, 10 im Kissen 8 hin und her, also zu den Schultern 7 hin oder davon fort bewegen läßt, kann dieser Schulterbräuner an die Leistung der Sonnenbank angepaßt werden.

Der aktive Hochdruckschulterbräuner besteht aus einer in das Kopfkissen 8 eingearbeiteten Leuchteinheit, aus Strahler 2, Reflektor 3 und nachgeschaltetem Filter 15, einem V-förmigen Umlenkspiegel 16 und entweder einer Streuebene 18 und/oder einem auf die Schulter 7 gerichteten Reflektor 17.

Der V-förmige Umlenkspiegel 16 teilt das aus der Leuchteinheit nach hinten austretende Bräunungslicht 19 in zwei gleiche Strahlungen und führt diese zu den beiden Seiten heraus.

Hieran schließt sich eine Streuebene 18 an, mittels welcher die Schultern 7 gleichmäßig mit UV-Licht bestrahlt werden und das IR-Licht an den Schultern 7 vorbeigeleitet wird.

Das vom Umlenkspiegel auf einen weiteren Reflektor 17 geleitete Nutzlicht wird auf die Schulterpartie 7 geleitet, wo es für eine gleichmäßige Bestrahlung sorgt.

Bei den aktiven Einheiten sind die nötigen Vorschalt- und Zündgeräte in das Kopfkissen eingebaut, so daß diese Einheiten völlig unabhängig von der Sonnenbank arbeiten. Auf diese Weise läßt sich jede Sonnenbank entweder mit einem passiven oder auch mit einem aktiven Schulterbräuner nachrüsten.

## Patentansprüche

1. Bräunungsvorrichtung zur Anwendung in Sonnenbänken mit einem Gesichtsbräuner (2, 3), **dadurch gekennzeichnet**, daß im Kopf-/Schulterbereich (7) des Benutzers (1) ein Kissen (8) mit einem Reflektorkragen (5) vorgesehen ist, dessen verspiegelte Oberfläche (6) gekrümmt ist, wobei das Lot senkrecht auf seinen Oberflächenelementen (6) jeweils Winkelhalbierende des Winkels ist, der gebildet wird aus dem aus dem Gesichtsbräuner (2, 3) austretenden Strahl (4) und dem auf die Schulter (7) auftreffenden Strahl.

2. Bräunungsvorrichtung zur Anwendung in Sonnenbänken, **dadurch gekennzeichnet**, daß im Kopf-/Schulterbereich (7) des Benutzers (1) ein Kissen vorgesehen ist, daß im Kissen ein Schacht (11) ausgebildet ist, in dem ein Verbindungsgestell (13) angeordnet ist, daß sich beidseitig an das Verbindungsgestell (13) je eine Sockelhalterung (12) mit einseitig gesockelten Lampen (9) anschließt und daß hinter den Lampen (9) jeweils ein sich zu den Schultern (7) öffnender Reflektor (10) angeordnet ist.

3. Bräunungsvorrichtung nach Anspruch 2, **dadurch gekennzeichnet**, daß das Verbindungsgestell (13) relativ zur Schulter (7) des Benutzers (1) bewegbar angeordnet ist.

4. Bräunungsvorrichtung nach Anspruch 2 oder 3, **dadurch gekennzeichnet**, daß die Lampen (9) als UV-Niederdruckstrahler ausgebildet sind.

5. Bräunungsvorrichtung zur Anwendung in Sonnenbänken, **dadurch gekennzeichnet**, daß im Kopf-/Schulterbereich (7) des Benutzers (1) ein Kissen (8) mit einer Leuchteinheit vorgesehen ist, die aus einem Strahler (2), einem ersten Reflektor (3) und einem Filter (15) besteht, wobei die reflektierte und gefilterte Strahlung über Umlenkspiegel (16) auf eine Streuebene (18) und/oder einen zweiten Reflektor (17) und von dort zum Schulterbereich (7) geführt wird.

6. Bräunungsvorrichtung nach Anspruch 5, **dadurch gekennzeichnet**, daß der Strahler (2) als UV-Hochdruckstrahler ausgebildet ist.

7. Bräunungsvorrichtung nach einem der Ansprüche 2 bis 6, **dadurch gekennzeichnet**, daß die zum Betrieb der die Bräunungsstrahlung emittierenden Strahlenquelle notwendigen Vorschalt- und Zündgeräte innerhalb des Schulterkissens (8) angeordnet sind.

8. Bräunungsvorrichtung nach einem der Ansprüche 2 bis 7, **dadurch gekennzeichnet**, daß für die zur Kühlung der Strahlenquelle, der Vorschalt- oder der Zündgeräte notwendigen Kühlvorrichtungen innerhalb des Schulterkissens (8) angeordnet sind.

9. Vorrichtung nach einem der Ansprüche 2 bis 8, **dadurch gekennzeichnet**, daß das Schulterkissen (8) zusätzlich einen Reflektor nach Anspruch 1 aufweist.

10. Bräunungsvorrichtung zur Anwendung in Sonnenbänken, **dadurch gekennzeichnet**, daß hinter der Schulter (7) eines Benutzers (1) in einem Kopfkissen ein die Schulter (7) mit UV-Licht beaufschlagendes Strahlungssystem vorgesehen ist.

## Claims

1. Tanning device for use in sun beds, with a facial tanner (2,3), characterized in that there is provided, in the head/shoulder area (7) of the user (1), a cushion (8) with a reflector collar (5), the mirrored surface (6) of which collar is curved, wherein the line perpendicular to the surface elements (6) of the collar bisects, in each case, the angle which is formed of the ray (4) exiting from the facial tanner (2,3) and the ray which arrives on the shoulder (7).

2. Tanning device for use in sun beds, characterized in that a cushion is provided in the head/shoulder area (7) of the user (1); in that a shaft (11) is formed in the cushion, in which shaft a connection frame (13) is disposed; in that, in each case, a socket mounting (12) with lamps (9) which are equipped on one side with sockets is connected to a connection frame (13) on both sides; and in that a reflector (10) which widens towards the shoulders (7) is disposed behind the lamps (9) in each case.

3. Tanning device according to Claim 2, characterized in that the connection frame (13) is disposed in a moveable manner in relation to the shoulder (7) of the user (1).

4. Tanning device according to Claim 2 or 3, characterised in that the lamps (9) are formed as UV low pressure radiators.

5. Tanning device for use in sun beds, characterised in that a cushion (8) with a luminescent unit is provided in the head/shoulder area (7) of the user (1), which unit consists of a radiator (2), a first reflector (3) and a filter (15), wherein the reflected and filtered radiation is conducted, via deviation mirrors (16), onto a dispersion plane (18) and/or onto a second reflector (17) and is conducted from there to the shoulder area (7).

6. Tanning device according to Claim 5, characterised in that the radiator (2) is formed as a UV high pressure radiator.

7. Tanning device according to any one of Claims 2 to 6, characterised in that the power supply and ignition devices which are necessary for the operation of the ray source which emits the tanning radiation are disposed inside the shoulder cushion (8).

8. Tanning device according to any one of Claims 2 to 7, characterised in that the cooling devices, which are necessary for cooling the ray source, the power supply and ignition devices, are disposed inside the shoulder cushion (8).

9. Device according to one of Claims 2 to 8, characterised in that the shoulder cushion (8) comprises, additionally, a reflector according to Claim 1.

10. Tanning device for use in sun beds, characterised in that a radiation system which acts on the shoulder (7) with UV light is provided behind the shoulder (7) of a user (1) in a pillow.

## Revendications

1. Dispositif de bronzage destiné à être utilisé dans des solariums équipés d'un bronzeur du visage (2,3), caractérisé en ce qu'il est prévu, dans la région (7) de la tête et des épaules de l'utilisateur (1), un coussin (8) muni d'une collerette réflectrice (5) dont la surface rendue réfléchissante (6) est courbe, la perpendiculaire à chacun de ses éléments de surface (6) étant la bissectrice de l'angle qui est formé entre le rayon lumineux (4) issu du bronzeur de visage (2,3) et le rayon incident sur l'épaule (7).

2. Dispositif de bronzage destiné à être utilisé dans des solariums, caractérisé en ce qu'un coussin est prévu dans la région (7) de la tête et des épaules de l'utilisateur (1), en ce qu'il est formé, dans le coussin, un puits (11) dans lequel est disposé un bâti de jonction (13), en ce qu'un support de douilles (12) équipé de lampes (9) à culot unilatéral est raccordé au bâti de jonction (13) de chacun des deux côtés de celui-ci, et en ce qu'un réflecteur (10) qui s'ouvre vers les épaules (7) est disposé de chaque côté en arrière des lampes (9).

3. Dispositif de bronzage selon la revendication 2, caractérisé en ce que le bâti de jonction (13) est monté mobile par rapport à l'épaule (7) de l'utilisateur (1).

4. Dispositif de bronzage selon la revendication 2 ou 3, caractérisé en ce que les lampes (9) sont réalisées sous forme de lampes à rayons UV à basse pression.

5. Dispositif de bronzage destiné à être utilisé dans des solariums, caractérisé en ce qu'il est prévu, dans la région (7) de la tête et des épaules de l'utilisateur (1), un coussin (8) avec un groupe lumineux qui se compose d'une source de rayonnement (2), d'un premier réflecteur (3) et d'un filtre (15), le rayonnement réfléchi et filtré étant dirigé, au moyen de miroirs de déviation (16), sur un plan de diffusion (18) et/ou sur un second réflecteur (17) et, de là, vers la région des épaules (7).

6. Dispositif de bronzage selon la revendication 5, caractérisé en ce que l'émetteur de rayonnement (2) est réalisé sous forme d'émetteur de rayons UV à haute pression.

7. Dispositif de bronzage selon l'une quelconque des revendications 2 à 6, caractérisé en ce que les appareils de régulation de puissance et d'allumage, nécessaires pour le fonctionnement de la source de rayonnement qui émet le rayonnement de bronzage, sont disposés à l'intérieur du coussin d'épaules (8).

8. Dispositif de bronzage selon l'une quelconque des revendications 2 à 7, caractérisé en ce que les dispositifs de refroidissement nécessaires pour le refroidissement de la source de rayonnement et des appareils de régulation de puissance ou d'allumage sont disposés a l'intérieur du coussin d'épaules (8).

9. Dispositif de bronzage selon l'une quelconque des revendications 2 à 8, caractérisé en ce que le coussin d'épaules (8) comporte en plus un réflecteur selon la revendication 1.

10. Dispositif de bronzage destiné à être utilisé dans des solariums, caractérisé en ce qu'il est prévu, en arrière de l'épaule (7) d'un utilisateur (1), dans un oreiller, un système émetteur de rayonnement qui projette de la lumière UV sur l'épaule (7).
